(19) Europäisches Patentamt
European Patent Office
Office européen des brevets

(11) EP 3 461 403 A1

(12) **EUROPEAN PATENT APPLICATION**

(43) Date of publication:
03.04.2019 Bulletin 2019/14

(51) Int Cl.:
*A61B 5/11* (2006.01)   *A61B 5/00* (2006.01)

(21) Application number: 17194125.5

(22) Date of filing: 29.09.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Koninklijke Philips N.V.
5656 AE Eindhoven (NL)

(72) Inventors:
• SAPORITO, Salvatore
5656 AE Eindhoven (NL)

• ANNEGARN, Janneke
5656 AE Eindhoven (NL)
• RISPENS, Sietse Menno
5656 AE Eindhoven (NL)
• SPINA, Gabriele
5656 AE Eindhoven (NL)

(74) Representative: de Haan, Poul Erik
Philips International B.V.
Philips Intellectual Property & Standards
High Tech Campus 5
5656 AE Eindhoven (NL)

(54) **A METHOD AND APPARATUS FOR ASSESSING THE MOBILITY OF A SUBJECT**

(57) According to an aspect, there is provided an apparatus (2) for assessing the mobility of a subject, the apparatus (2) comprising a processing unit (4) that is configured to obtain measurements of the movements of the subject over time; process the obtained measurements of the movements to determine values for a first plurality of parameters; and combine the determined values for the first plurality of parameters to determine an estimate of the time that the subject would take to complete a timed up-and-go, TUG, test.

Figure 1

**Description**

TECHNICAL FIELD OF THE INVENTION

The invention relates to a method and apparatus for assessing the mobility of a subject.

BACKGROUND TO THE INVENTION

Functional mobility is a term used to reflect the balance and gait performances in everyday life of a person. Mobility impairment is common in the elderly population, and it impacts the physical, psychological, and social wellness of older adults. The assessment of mobility is, therefore, important in assessing the condition of a person, and in particular frail elderly individuals.

Mobility is commonly evaluated by assessing a person's ability in activities performed in daily life, such as transferring from a bed or chair, walking a certain distance, or climbing stairs independently. Mobilisation (i.e. getting the person mobile) is a key component of rehabilitation therapy after hospitalisation for surgery or acute illness.

Standardised functional tests are often used to assess functional mobility. In particular, the timed up and go (TUG) is a well-accepted and established functional mobility screening test. The TUG test requires a subject to get up from a chair, walk 3 metres, turn around, return to the seat and sit down. The time taken to perform the test is considered a useful performance measure, with a longer duration being associated with decreased mobility/increased frailty.

The TUG test tests sit-to-stand and stand-to-sit chair transfer tasks, turning, walking in a straight line, balance control and the ability to follow a sequence of tasks. As such, the TUG test assesses several mobility components such as power, agility, and balance. TUG scores (i.e. TUG times) have been found to correlate well with performance levels in more extensive and time-consuming tests for balance as well as with gait speed.

Functional mobility will vary over time, for example due to aging, injury, illness, in response to interventions such as exercise programs or medication, or during post-surgery rehabilitation, and therefore it is important to measure functional mobility regularly to monitor progress or decline in a subject, particularly an elderly subject.

However, TUG and other standardised tests are normally carried out under expert supervision by a clinician or a physiotherapist, for example in a clinic or hospital setting. Due to the time and resources required by such a procedure, the TUG test cannot be performed frequently in practice, and often it is administered only at one moment in time (i.e. at the clinic), limiting its applicability as a general screening tool.

Mobility assessment using wearable sensors (for example accelerometers) can allow for a low-cost, unobtrusive alternative to tests in laboratory or clinical settings, particularly those that require dedicated test equipment. Mobility assessment using wearable sensors might not require supervision or trained personnel, and an evaluation of a subject can take place continuously, allowing the evaluation of trends in mobility over time without the need for periodic visits to a clinic or laboratory. An assessment that takes place during unsupervised or unstructured daily routines can provide an indication of individual mobility in free-living conditions.

However, a drawback with the assessment of activities in daily living is that there is a wide range of activities that could be detected in sensor measurements and a wide range of parameters that can be derived for or from those activities and used to assess mobility or functional mobility. While simple measures such as step count have already been proposed as measures of functional ambulatory status, there are widely accepted metrics or parameters that provide a more comprehensive measure of mobility, like the time taken to complete a TUG test.

Therefore there is a need for an alternative way to monitor the mobility of a subject during normal and/or unstructured physical activities to provide a measure of the mobility of the subject.

SUMMARY OF THE INVENTION

According to a first aspect, there is provided an apparatus for assessing the mobility of a subject (e.g. a person, an individual, a patient, a user), the apparatus comprising a processing unit that is configured to obtain measurements of movements of the subject over time; process the obtained measurements to determine values for a first plurality of parameters; and combine the determined values for the first plurality of parameters to determine an estimate of the time that the subject would take to complete a TUG test.

Thus, this aspect provides a way to obtain a recognised mobility measure, namely the time taken to complete a TUG test, from measurements of the movements of the subject.

In some embodiments, the processing unit is configured to obtain the measurements of the movements from a memory unit or database. In this way the measurements are not required to be processed in real time and can be processed as and when it is desired to assess the mobility of the subject.

In alternative embodiments, the processing unit is configured to obtain the measurements of the movements from one or more sensors. In this way the mobility of the subject can be assessed in real-time or near real-time.

In some embodiments, the measurements of the movements are provided by one or more sensors, for example one or more of an accelerometer, gyroscope, air pressure sensor and position sensor.

In some embodiments, the measurements of the movements of the subject are measurements of the movements of the subject during activities of daily living of the subject. This has the advantage that the subject is not required to perform any specific activity or exercise in order for the TUG time estimate to be derived.

In some embodiments, the first plurality of parameters relate to any one or more of walking, sit-to-stand transitions, stand-to-sit transitions, walking on stairs, physical activity levels and sedentary periods.

In some embodiments, the processing unit is configured to combine the values for the first plurality of parameters according to a model to determine the estimate of the time that the subject would take to complete a TUG test.

In some embodiments, the processing unit is further configured to determine the model by obtaining measurements of the movements of a plurality of people over time; obtaining measurements of the times taken for the plurality of people to complete the TUG test; processing the obtained measurements of the movements of the plurality of people and obtained measurements of the times taken for the plurality of people to complete the TUG test to determine values for a second plurality of parameters; evaluating the values for the second plurality of parameters to identify a first subset of the second plurality of parameters that are statistically dependent on the obtained measurements of the times taken for the plurality of people to complete the TUG test, wherein the first subset of the second plurality of parameters is the first plurality of parameters; and deriving the model for determining an estimate of the time that a subject would take to complete the TUG test based on the first subset of the second plurality of parameters. This embodiment provides the advantage that the range of possible parameters that could be measured or extracted for a subject can be refined to provide parameters that are useful for determining the TUG time estimate.

In some embodiments, the processing unit is further configured to evaluate the values for the second plurality of parameters by determining a first measure of statistical dependence for each of the parameters in the second plurality of parameters based on a measure of association between the values for each of the second plurality of parameters and the time taken for the plurality of people to complete the TUG test; and selecting the first subset of the second plurality of parameters as (i) the parameters having a measure of statistical dependence above a threshold value; or (ii) a predetermined number of parameters having the highest measures of statistical dependence. In this way only the most relevant parameters, or a predetermined number of most relevant parameters are selected for the model, which reduces the number of parameters in the model and thus reduces the processing of the movement measurements required when the TUG time estimate is to be determined.

In some embodiments, the processing unit is further configured to evaluate the values for the second plurality of parameters by determining a first measure of statistical dependence for each of the parameters in the second plurality of parameters based on a statistical dependence between the values for each of the second plurality of parameters and the time taken for the plurality of people to complete the TUG test; selecting a second subset of the second plurality of parameters as the parameters having a first measure of statistical dependence above a first threshold value; determining a second measure of statistical dependence for each of the parameters in the second subset of the second plurality of parameters based on a measure of association between the values for each of parameters in the second subset of the second plurality of parameters with each of the other parameters in the second subset of the second plurality of parameters; for any pair of parameters in the second subset of the second plurality of parameters having a second measure of statistical dependence above a second threshold value, discarding from the second subset the one of the pair of parameters having the lowest first measure of statistical dependence; and forming the first subset of the second plurality of parameters based on the remaining ones of the second subset of the second plurality of parameters. In this way only the most relevant parameters are selected for the model, with any 'redundant' parameters that exhibit a similar relationship with TUG time to other parameters being discarded, which thereby reduces the number of parameters in the model and thus reduces the processing of the movement measurements required when the TUG time estimate is to be determined.

In some embodiments, the first measure of statistical dependence is a Spearman's rank correlation coefficient, SCC.

In some embodiments, the processing unit is configured to derive the model using a regularised linear model.

In some embodiments, the processing unit is configured to derive parameters $\tilde{\beta}, \tilde{\beta}_0$ for the model by minimising

the cost $\min_{\beta, \beta_0} \sum_{i=subjects} (y_i - \beta_0 - x_i \beta)^2 + \lambda \|\beta\|_1$ where $y_i$ is the time taken for a TUG test for the i-th person, $\beta$ and $\beta_0$ are regression coefficients for the $x_i$ parameter in the first subset of the second plurality of parameters, and $\lambda$ is the model regulariser.

In some embodiments, the apparatus further comprises one or more sensors for measuring movements of the subject over time, and the processing unit is configured to obtain the measurements of the movements of the subject from the one or more sensors.

According to a second aspect, there is provided a system for assessing the mobility of a subject, the system comprising a sensor unit comprising one or more sensors for measuring movements of the subject over time, with the sensor unit being configured to generate a sensor signal based on said measured movements of the subject over time; and an apparatus as defined in the first aspect or any embodiment thereof, with the processing unit being configured to obtain the measurements of the movements of the subject by receiving the sensor signal from the sensor unit.

According to a third aspect, there is provided a system for assessing the mobility of a subject, the system comprising a sensor unit comprising one or more sensors for measuring movements of the subject over time, with the sensor unit being configured to generate a sensor signal based on said measured movements of the subject over time; and an apparatus comprising a processing unit that is configured to receive the sensor signal from the sensor unit; process the sensor signal to determine values for a first plurality of parameters; and combine the determined values for the first plurality of parameters to determine an estimate of the time that the subject would take to complete a TUG test.

According to a fourth aspect, there is provided a method of assessing the mobility of a subject, the method in a processing unit comprising obtaining measurements of the movements of the subject over time; processing the obtained measurements of the movements to determine values for a first plurality of parameters; and combining the determined values for the first plurality of parameters to determine an estimate of the time that the subject would take to complete a TUG test. Thus, this aspect provides a way to obtain a recognised mobility measure, namely the time taken to complete a TUG test, from measurements of the movements of the subject.

In some embodiments, the step of obtaining comprises obtaining the measurements of the movements from a memory unit or database. In this way the measurements are not required to be processed in real time and can be processed as and when it is desired to assess the mobility of the subject.

In alternative embodiments, the step of obtaining comprises obtaining the measurements of the movements from one or more sensors. In this way the mobility of the subject can be assessed in real-time or near real-time.

In some embodiments, the measurements of the movements are provided by one or more sensors, for example one or more of an accelerometer, gyroscope, air pressure sensor and position sensor.

In some embodiments, the measurements of the movements of the subject are measurements of the movements of the subject during activities of daily living of the subject. This has the advantage that the subject is not required to perform any specific activity or exercise in order for the TUG time estimate to be derived.

In some embodiments, the first plurality of parameters relate to any one or more of walking, sit-to-stand transitions, stand-to-sit transitions, walking on stairs, physical activity levels and sedentary periods.

In some embodiments, the step of combining comprises combining the values for the first plurality of parameters according to a model to determine the estimate of the time that the subject would take to complete a TUG test.

In some embodiments, the method further comprises the step of determining the model. The model can be determined by obtaining measurements of the movements of a plurality of people over time; obtaining measurements of the times taken for the plurality of people to complete the TUG test; processing the obtained measurements of the movements of the plurality of people and obtained measurements of the times taken for the plurality of people to complete the TUG test to determine values for a second plurality of parameters; evaluating the values for the second plurality of parameters to identify a first subset of the second plurality of parameters that are statistically dependent on the obtained measurements of the times taken for the plurality of people to complete the TUG test, wherein the first subset of the second plurality of parameters is the first plurality of parameters; and deriving the model for determining an estimate of the time that a subject would take to complete the TUG test based on the first subset of the second plurality of parameters. This embodiment provides the advantage that the range of possible parameters that could be measured or extracted for a subject can be refined to provide parameters that are useful for determining the TUG time estimate.

In some embodiments, the step of evaluating further comprises evaluating the values for the second plurality of parameters by determining a first measure of statistical dependence for each of the parameters in the second plurality of parameters based on a measure of association between the values for each of the second plurality of parameters and the time taken for the plurality of people to complete the TUG test; and selecting the first subset of the second plurality of parameters as (i) the parameters having a measure of statistical dependence above a threshold value; or (ii) a predetermined number of parameters having the highest measures of statistical dependence. In this way only the most relevant parameters, or a predetermined number of most relevant parameters are selected for the model, which reduces the number of parameters in the model and thus reduces the processing of the movement measurements required when the TUG time estimate is to be determined.

In some embodiments, the step of evaluating further comprises evaluating the values for the second plurality of parameters by determining a first measure of statistical dependence for each of the parameters in the second plurality of parameters based on a statistical dependence between the values for each of the second plurality of parameters and the time taken for the plurality of people to complete the TUG test; selecting a second subset of the second plurality of parameters as the parameters having a first measure of statistical dependence above a first threshold value; determining a second measure of statistical dependence for each of the parameters in the second subset of the second plurality of parameters based on a measure of association between the values for each of parameters in the second subset of the second plurality of parameters with each of the other parameters in the second subset of the second plurality of parameters; for any pair of parameters in the second subset of the second plurality of parameters having a second measure of statistical dependence above a second threshold value, discarding from the second subset the one of the pair of parameters having the lowest first measure of statistical dependence; and forming the first subset of the second plurality of parameters based on the remaining ones of the second subset of the second plurality of parameters. In this way only the most relevant parameters are selected for the model, with any 'redundant' parameters that exhibit a similar relationship with TUG time to other parameters being discarded, which thereby reduces the number of parameters in the model and thus reduces the processing of the movement measurements required when the TUG time estimate is to be determined.

In some embodiments, the first measure of statistical dependence is a Spearman's rank correlation coefficient, SCC.

In some embodiments, the step of deriving the model comprises deriving the model using a regularised linear model.

In some embodiments, the step of deriving the model comprises deriving parameters $\tilde{\beta}, \tilde{\beta}_0$ for the model by

$$\min_{\beta, \beta_0} \sum_{i=subjects} (y_i - \beta_0 - \boldsymbol{x}_i \boldsymbol{\beta})^2 + \lambda \|\boldsymbol{\beta}\|_1$$

minimising the cost where $y_i$ is the time taken for a TUG test for the i-th person, $\beta$ and $\beta_0$ are regression coefficients for the $\boldsymbol{x}_i$ parameter in the first subset of the second plurality of parameters, and $\lambda$ is the model regulariser.

According to a fifth aspect, there is provided a computer program product comprising a computer readable medium having computer readable code embodied therein, the computer readable code being configured such that, on execution by a suitable computer or processor, the computer or processor is caused to perform the method according to the third aspect or any embodiment thereof.

These and other aspects of the invention will be apparent from and elucidated with reference to the embodiment(s) described hereinafter.

BRIEF DESCRIPTION OF THE DRAWINGS

Exemplary embodiments of the invention will now be described, by way of example only, with reference to the following drawings, in which:

Figure 1 is a block diagram of an apparatus according to an embodiment and a sensor unit;

Figure 2 is a flow chart illustrating a method according to an embodiment;

Figure 3 is a flow chart illustrating a method of determining a model according to an embodiment;

Figure 4 is a set of graphs showing various movement parameters against TUG time;

Figure 5 is a graph illustrating the output of an exemplary model over for several subjects; and

Figure 6 is a flow chart illustrating a method of identifying a subset of parameters according to an exemplary embodiment.

DETAILED DESCRIPTION OF THE PREFERRED EMBODIMENTS

As noted above, mobility assessment using wearable sensors (for example accelerometers) can allow the mobility of a subject to be monitored during their normal daily activities as an alternative to tests in laboratory or clinical settings. However, the range of different types of movements and the range of different parameters for those types of movements that can be measured means that it is difficult to provide a consistent and widely understood measure of the mobility of the subject. Thus, the invention provides that a number of different parameters derived from measurements of the movements of a subject are combined to provide an estimate of the time that the subject would take to complete a timed-up-and-go (TUG) test.

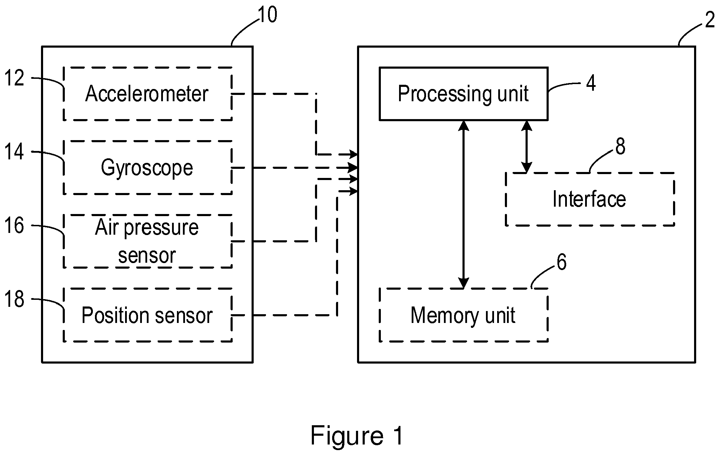

Figure 1 shows an apparatus 2 for assessing the mobility of a subject according to an embodiment. The apparatus 2 comprises a processing unit 4 that controls the operation of the apparatus 2 and generally implements the method according to the invention. The processing unit 4 can be implemented in numerous ways, with software and/or hardware, to perform the various functions described below. The processing unit 4 may comprise one or more microprocessors or digital signal processor (DSPs) that may be programmed using software or computer program code to perform the required functions and/or to control components of the processing unit 4 to effect the required functions.

The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

In various implementations, the processing unit 4 may be associated with or comprise one or more memory units 6 such as volatile and non-volatile computer memory such as RAM, PROM, EPROM, and EEPROM. The processing unit 4 or associated memory unit 6 can also be used for storing program code that can be executed by a processor in the processing unit 4 to perform the methods described herein. In some embodiments, the memory unit 6 can store information generated during execution of the methods, information for use during execution of the methods (e.g. measurements from one or more sensors, a model or algorithm used to combine sensor measurements to determine an estimate of the time that the subject would take to complete a TUG test or the output(s) of the methods (e.g. the estimates of the time that the subject would take to complete a TUG test). The term "TUG time estimate" is used herein to refer to the estimate of the time that the subject would take to complete a TUG test.

The apparatus 2 also comprises an interface 8 for outputting the TUG time estimate. The interface 8 may be a user interface for outputting the TUG time estimate determined by the processing unit 4 to a user of the apparatus 2. Depending on the way in which the apparatus 2 is implemented, the user of the apparatus 2 may be the subject themselves (i.e. the subject being monitored), or a clinician or care provider. The user interface 8 can therefore be or comprise a display screen or one or more other elements (e.g. lights or LEDs) for providing a visual output, a speaker for providing an audible output, a vibrating element for providing a tactile output, or any combination thereof. The user interface 8 may also enable the user to interact with the apparatus 2, for example to activate or deactivate the apparatus 2 and/or to control one or more settings or operations of the apparatus 2. As such the interface 8 can comprise any one or more of a touch screen, button(s), switch(es), keypad, keyboard, mouse, trackpad, stylus, etc. As an alternative or in addition to a user interface, the interface 8 may be an interface for allowing the apparatus 2 to exchange information with another electronic device (e.g. a computer or server). In that case the interface 8 may enable the apparatus 2 to establish a wired or wireless connection with the other electronic device using any suitable communication protocol or communication technology (for example USB, Ethernet, Zigbee, Bluetooth, WiFi, a wide-area network technology, a cellular telecommunication technology such as Global System for Mobile communications (GSM), Universal Mobile Telecommunications Service (UMTS) or Long Term Evolution (LTE), etc.).

In order for the processing unit 4 to derive a number of different parameters from measurements of the movements of a subject and combine them to provide an estimate of the time that the subject would take to complete a TUG test, measurements of the movements of the subject are required. Figure 1 shows a sensor unit 10 that comprises one or more sensors for measuring the movement of the subject over time.

In some embodiments (e.g. as shown in Figure 1), the apparatus 2 and sensor unit 10 form a system for assessing the mobility of the subject in which the sensor unit 10 is separate from the apparatus 2, and the measurements are provided from the sensor unit 10 to the apparatus 2, for example via the interface 8. The measurements can be provided by the sensor unit 10 to the apparatus 2 in the form of a sensor signal that is generated based on or from the movement measurements. The measurements (i.e. the sensor signal) can be provided to the apparatus 2 in real-time, or the sensor unit 10 can periodically or intermittently provide the measurements (sensor signal) to the apparatus 2. In some embodiments, the measurements from the sensor unit 10 can be stored in a memory unit or database, and the apparatus 2 can obtain or retrieve the measurements from the memory unit or database when a TUG time estimate is to be determined.

The one or more sensors in the sensor unit 10 can include any sensor for measuring the movements of a subject, and can include any one or more of an accelerometer 12, a gyroscope 14, an air pressure sensor 16 for measuring air pressure (that can be used to identify changes in altitude of the subject, for example walking up or down stairs), and a position or location sensor 18, such as a satellite positioning system receiver (e.g. GPS, Galileo, etc.). Those skilled in the art will be aware of other types of sensors that can be used.

In some embodiments all of the components, e.g. the processing unit 4, memory unit 6, interface 8, sensor unit 10, sensor(s) 12, 14, 16, 18 (if present), can be contained within the apparatus 2. For example the apparatus 2 could be in the form of a smartphone, tablet computer, pendant, bracelet, smart watch, etc. that can be carried or worn by the subject. In alternative embodiments, the sensor unit 10 comprising the one or more sensor(s) 12, 14, 16, 18 is in the form of a wearable or portable device, such as a smartphone, tablet computer, pendant, bracelet, smart watch, etc., and the apparatus 2 is separate from the sensor unit 10. In that case the apparatus 2 may be local to the sensor unit 10, for example in wired or wireless communication with the sensor unit 10, or it may be remote from the sensor unit 10. For example the apparatus 2 could be, or be part of, a computer, server, laptop, tablet computer, etc. In some embodiments, the functions of the apparatus 2 could be implemented by a distributed arrangement of computers/servers.

It will be appreciated that the apparatus 2 may comprise additional components to those shown in Figure 1.

For example the apparatus 2 may comprise a power source, such as a battery, or a power interface component, such as plug, for connecting to the apparatus 2 to a mains power supply.

As described in more detail below, aspects of this disclosure relate to the derivation of a model that is used for determining the TUG time estimate from a plurality of parameters. In some embodiments, the apparatus 2 can be configured or adapted to derive the model, for example the processing unit 4 can be suitably configured or adapted, or the memory unit 6 can store suitable program code for execution by the processing unit 4 to derive the model. In other embodiments, the derivation of the model can be performed by a separate device or apparatus (e.g. a server or other computer), and the model sent or otherwise provided to the apparatus 2 for use in determining a TUG time estimate for a subject.

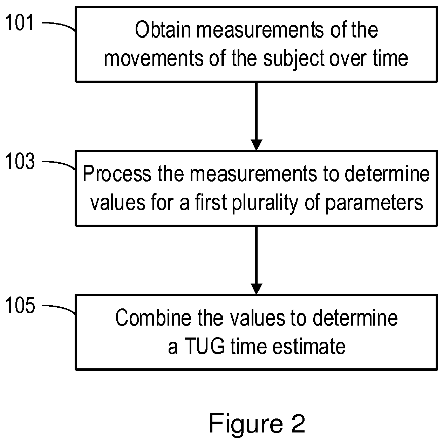

The flow chart in Figure 2 illustrates a method of assessing the mobility of a subject according to an aspect of the present disclosure. The method can be performed by the apparatus 2, for example by the processing unit 4 executing suitable program code stored in the memory unit 6 or received from an external source.

In a first step, step 101, measurements of the movements of the subject over time are obtained. This step can comprise receiving measurements from the sensor unit 10, for example in real-time or near real-time, or retrieving a set of measurements from memory unit 6 or an external database. The former case makes it possible for the apparatus 2 to determine a real-time or near-real-time TUG time estimate, i.e. based on the most recent measurements of the movements of the subject. However, generally the TUG time does not change significantly in a short period of time, so a TUG time estimate may only be estimated periodically or regularly, e.g. once per day, once per week, etc.

The measurements of the movements of the subject obtained in step 101 may be raw sensor measurements, e.g. a signal or set of samples as output by the sensor 12, 14, 16, 18, or the measurements of the movements may be pre-processed in some way, for example filtering to remove noise, etc.

The measurements of the movements of the subject are obtained during the normal daily activities of the subject (which can be referred to as the activities of daily living (ADL)). Normal daily activities can include walking, going up and down stairs, sleeping, sitting on a chair, etc., and are the activities that the subject performs or undertakes as part of their normal daily routine. For the purposes of this disclosure, tests or defined activities such as a TUG test or other specific exercise, activity or test designed to assess the mobility of the subject are not considered to be part of the activities of daily living. In some embodiments, the measurements are obtained continuously (subject only to a sampling frequency of the relevant sensor 12, 14, 16, 18), or they can be obtained periodically or regularly.

Next, in step 103, the measurements of the movements are processed to determine values for a first plurality of parameters. Each parameter relates to an aspect of mobility of the subject, and each parameter can relate to any one or more of walking, sit-to-stand transitions, stand-to-sit transitions, walking on stairs (including walking up and/or down stairs), physical activity levels, sedentary (inactive) periods (i.e. periods where the subject is resting or at rest), the environment in which the subject is walking/moving (e.g. indoors or outdoors), walking on a particular type of terrain or ground, whether the subject is walking with or without shoes or with or without a walking aid, and localisation data (e.g. a total life space of the subject). The first plurality of parameters may comprise any desired number of parameters, for example, 2, 10, 20, 30, etc.

Those skilled in the art will be aware of parameters related to the mobility of a subject that can be determined from measurements of the movements of a subject, particularly where those measurements are obtained using one or more of an accelerometer 12, gyroscope 14, air pressure sensor 16 and/or position sensor 18, and thus a full list is not provided herein. However, suitable parameters include any one or more of step count (i.e. the number of footsteps in a time period), a step rate (i.e. the number of footsteps per unit time), step time (i.e. the time between footsteps when walking), step/gait regularity (i.e. a measure of how regular the footsteps are), a walking speed, a maximum walking speed, the time taken to stand up from a sitting position on a chair, the time taken to sit down on a chair from a standing position, the time taken to go up a set of stairs, the time taken to go down a set of stairs, a maximum physical activity level, the duration of sedentary periods, etc. Suitable parameters can also or alternatively include any of an average (including any of mode, median, percentiles and/or mean) of any one or more (or combination) of the aforementioned (or other) parameters, a variance of any one or more (or combination) of the aforementioned (or other) parameters, a shape of a distribution of values of any one or more (or combination) of the aforementioned (or other) parameters (e.g. skewness, kurtosis), parameters derived by frequency domain decomposition of the measurements of the movements, parameters derived by phase portrait representation derived from measurements of the movements, a measure of consistency of any one or more (or combination) the aforementioned (or other) parameters (e.g. reliability coefficients), etc. In some embodiments, values for one or more of these parameters can be determined from multiple instances of the movement. For example, a walking speed can be obtained by evaluating several different walking episodes by the subject (which can occur on the same or different days). Likewise, the time taken to go down a set of stairs may be determined by evaluating the sensor measurements obtained during several different stair descents. In some embodiments, a parameter value can be obtained for multiple events that are aggregated over a time period, such as daily, and subsequently combined (e.g. averaged) over a longer time period (e.g. several days) to provide a value for the parameter for the longer time period (e.g. a week).

Next, in step 105, the values for the first plurality of parameters are combined to determine an estimate of the time that the subject would take to complete the TUG test (i.e. determine the TUG time estimate). In this way, the described method provides that a TUG time estimate can be derived from measurements of the movements of a subject obtained during the normal daily activities of the subject, i.e. without requiring the subject to perform a TUG test or other specific exercise, activity or test. Thus a TUG time estimate can be obtained unobtrusively.

Step 105 can comprise inputting the values for the first plurality of parameters into a model, such as an equation or function, that outputs a TUG time estimate. In some embodiments, the model can be used for all possible subjects (i.e. the same model is used for all subjects). In other embodiments, the model used in step 105 can be specific to the subject, or to a group of subjects that the subject is part of. For example, a specific model can be provided for subjects that have had a hip replacement, another model could be provided for subjects that have a particular medical condition that affects balance or mobility, etc. In some embodiments, the first plurality of parameters that are input into the model is the same for all possible subjects (i.e. the same parameters are used for all subjects). In other embodiments, the first plurality of parameters that are input to the model can be specific to the subject, or to a group of subjects that the subject is part of. For example, a specific set of parameters can be used for subjects that have had a hip replacement, and another set of parameters could be used for subjects that have a particular medical condition that affects balance or mobility, etc.

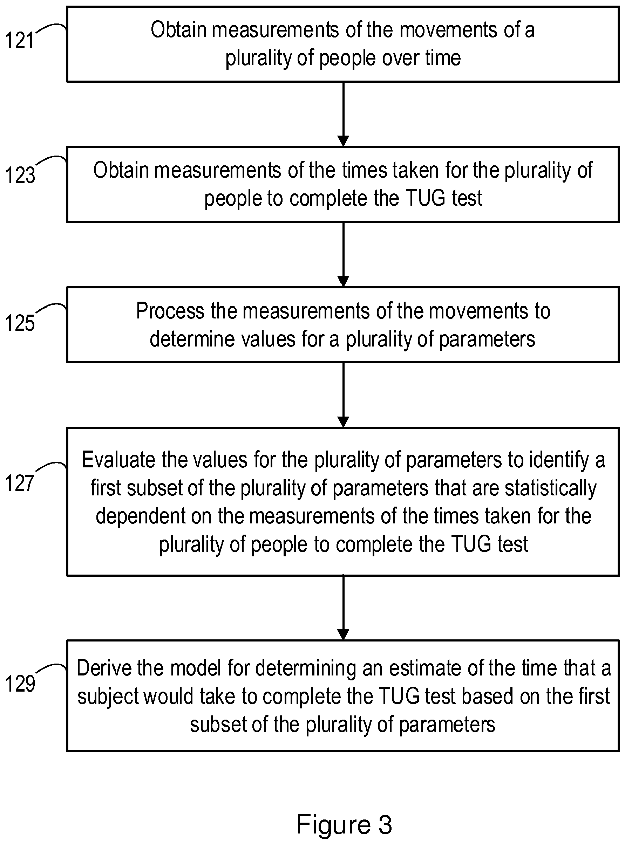

The flow chart in Figure 3 illustrates an exemplary method of determining a model that relates a plurality of parameters to a TUG time estimate. As noted above, the model can be determined by the apparatus 2, or it can be determined by a separate device or apparatus. The model is preferably determined prior to the steps in the method of Figure 2 being performed.

Briefly, the model is determined by identifying a set of relevant parameters (the first plurality of parameters) based on their associated with a TUG time in a population of subjects (particularly elderly subjects). The set of relevant parameters are used to derive a TUG time estimate by exploiting the observed relationship with TUG times.

In a first step, step 121, measurements of the movements of a plurality of people (e.g. elderly people) over time are obtained. In step 123, measurements of the times taken for the plurality of people to complete the TUG test (referred to as "TUG times") are obtained. The measurements in step 121 and/or step 123 can be obtained by retrieving them from a database or memory unit, or by receiving them directly from a respective sensor unit 10 (or similar device) worn or carried by each person. The TUG times are actual measurements of the TUG test as measured by a human observer (including the subject themselves) and/or by a dedicated test apparatus. The measurements obtained in steps 121 and 123 are to be used as a training set to derive the plurality of parameters and the model, and so the training set is preferably large, covering a large number of people (e.g. more than 50) and/or covering a relatively long period of time (e.g. at least several days).

Next, in step 125, the measurements of the movements and measurements of the TUG times are processed to determine values for a plurality of parameters. In particular, this step comprises determining values for all possible or all desirable parameters that could be considered for determining a TUG time estimate. A value for each parameter is determined for each person for which movement data is obtained. It should be noted that at this stage it is not known which, if any, parameters exhibit a relationship with TUG time.

In step 127, the values for the plurality of parameters are evaluated to identify a first subset of the plurality of parameters that are statistically dependent on TUG times. The first subset of parameters identified in step 127 are the parameters that are used to form the model, and are thus the parameters for which values are determined in step 103 above (in other words, the first subset of parameters is the first plurality of parameters described above).

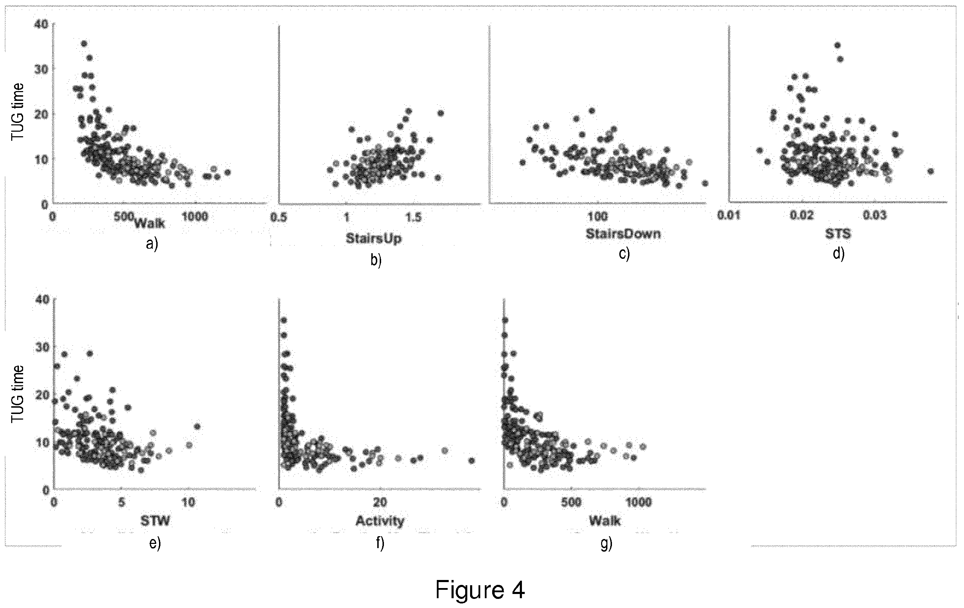

The graphs in Figure 4 show various movement-related parameters plotted against TUG time (i.e. the time actually taken to complete a TUG test) for a large number of people (approximately 200 people). Figure 4(a) shows a walking related parameter, namely the 90th daily percentile of walking spectral peak, against TUG time, and it can be seen that the walking parameter appears to exhibit an inverse relationship with TUG time. Figure 4(b) shows a parameter relating to moving up stairs, namely the 10th daily percentile of average stride time while walking up a slope, against TUG time, and there is no clear relationship between the value of the stairs parameter and TUG time. Figure 4(c) shows a parameter relating to moving down stairs, namely the 90th daily percentile of cadence while walking down a slope, against TUG time, and there is a suggestion of a linear relationship between the value of the stairs parameter and TUG time. Figure 4(d) shows a parameter relating to a sit-to-stand (STS) movement, namely the 25th daily percentile of sit to stand maximum jerk, against TUG time, and there is a suggestion of a weaker relationship between the value of the STS parameter and TUG time. Figure 4(e) shows a parameter relating to sit-to-walking (STW) (e.g. a sit-to-stand (STS) followed by walking), namely the 90th daily percentile of intensity of walk following a chair rise, against TUG time, and again there is a suggestion of a weaker relationship between the value of the STW parameter and TUG time. Figure 4(f) shows a parameter relating to activity, namely the maximum duration of time spent physically active (as opposed to sedentary) during the day at a given level, against TUG time, and there appears to be an inverse relationship between the value of the activity parameter and TUG time. Finally, Figure 4(g) shows another parameter relating to walking, namely the number of steps divided by the time the device was worn in a day, against TUG time, and again there appears to be an inverse relationship between the value of the walking parameter and TUG time.

In some implementations of step 127, a measure of the statistical dependence (e.g. a correlation coefficient) of each of the plurality of parameters with TUG times can be determined. In particular embodiments, the measure of the statistical dependence is a Spearman's rank correlation coefficient (SCC), but those skilled in the art will be aware of other measures of association that can be used. In some embodiments, the first subset of parameters can be selected as those parameters having a measure of statistical dependence above a threshold value. In one example, the threshold value for a Spearman correlation coefficient can be 0.4, although higher or lower values can be used. In other embodiments, the first subset of parameters can be selected as a predetermined number of parameters having the highest measure of statistical dependence. For example, the first subset of parameters can be the 10, 20, 30, etc. parameters having the highest measures of statistical dependence.

Depending on the parameter selection technique applied, based on the parameters shown in Figure 4, step 127 might identify the parameters in Figures 4(a), 4(c), 4(f) and 4(g) as being parameters that are statistically dependent on TUG times, and thus the first subset can comprise the parameters in Figures 4(a), 4(c), 4(f) and 4(g).

Next, in step 129, a model for determining a TUG time estimate is derived based on the first subset of parameters and their associated values for the plurality of people (and the TUG times for those people). Those skilled in the art will be aware of various ways or techniques for deriving a model from this training set. In specific embodiments, a regularised linear model is derived that relates the first subset of parameters to a TUG time estimate. In other embodiments, any regression technique could be used, such as, but not limited to, regression trees, support vector machines for regression, neural networks, generalised linear models, boosted gradient tree ensembles, random forests, etc.

In particular, parameters $\tilde{\beta}, \tilde{\beta}_0$ for a regularised linear model can be derived by minimising the cost:

$$\min_{\beta, \beta_0} \sum_{i=subjects} (y_i - \beta_0 - x_i \boldsymbol{\beta})^2 + \lambda \|\boldsymbol{\beta}\|_1 \qquad (1)$$

where $y_i$ is the time taken for a TUG test for the i-th person in the training set, $\beta$ and $\beta_0$ are regression coefficients for the $\boldsymbol{x}_i$ parameter in the first subset of parameters (i.e. the set of parameters derived in step 127, and $\lambda$ is the model regulariser. The model regulariser determines, together with the empirical data, the complexity of the model, i.e. how many parameters are included and how large the $\beta$ values are. The value of the model regulariser $\lambda$ can be automatically determined by, for example, inner 3-folds cross-validation within the training set. In some embodiments, the optimal $\lambda$ value can be selected as the one with the minimum average cross-validation squared error.

The performance of a resulting model can be evaluated on a training set (e.g. further measurements of the movements of a set of people/subjects) by performing a cross-validation.

If it is desired to provide a TUG time estimate even in the case where values for one or more of the parameters in the first subset have not or could not be determined in step 103 for a certain subject (for instance if a minimum number of events/activities/movements are not measured), a set of k models $\tilde{\beta}_k, \tilde{\beta}_{0_k}$ are trained for each of the available $\boldsymbol{x}_{i_k}$ feature subsets.

In order to determine a TUG time estimate for a new subject j, the measured parameter values can be averaged over a sliding window with a 3-day half-width centred around a day of interest, obtaining $\boldsymbol{x}_j$.

The model parameters $\tilde{\beta}_k \tilde{\beta}_{0_k}$ are used to derive the TUG time estimate; and the largest parameter subset k of the available parameters is used. The TUG time estimate is given by:

$$TUG\ time\ estimate = \widetilde{\boldsymbol{\beta}}_k x_j + \widetilde{\beta}_{0_k} \qquad (2)$$

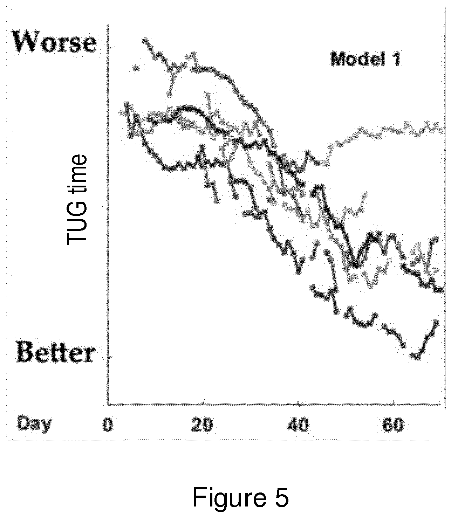

The graph in Figure 5 shows the outputs of the method of Figure 2 using a model derived according to the techniques described herein for seven different subjects. The subjects were evaluated over a period of 12 weeks during rehabilitation after a total hip replacement operation. It can be seen that the model shows almost continuous improvement in the estimated TUG time for almost all of the subjects, which is as expected for this group of subjects.

As noted above, in some embodiments the model derived in step 129 is used for all subjects/people. However, in other embodiments, separate models can be derived for different groups of subjects/people, such as those that have had a hip replacement or that have a particular medical condition. In that case, the method in Figure 3 can be repeated for each model to be derived, with the values for the parameters being derived from measurements of the movements of people in that group. This can mean that models for different groups of subjects/people can make use of different subsets of parameters.

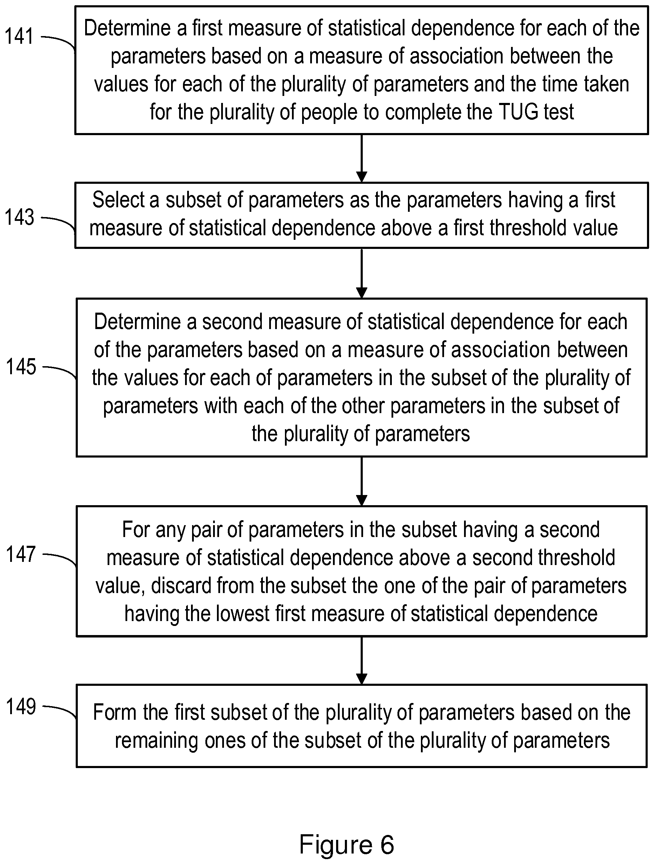

Since a very large number of parameters can be evaluated in step 125 (for example more than one hundred parameters), it is possible that there can be some redundancy between the parameters, and thus it is desired to remove redundant parameters, while retaining enough parameters and variation amongst the parameters to provide a reliable model for determining a TUG time estimate. The flow chart in Figure 6 illustrates a specific embodiment of step 127 in Figure 3, which is particularly useful where a very large number of parameters is evaluated in step 125.

In step 141, a first measure of statistical dependence (e.g. a correlation coefficient) is determined for each of the parameters in the plurality of parameters based on a measure of association (e.g. a correlation) between the parameter values and the associated TUG time. In particular embodiments, the measure of association is a Spearman's rank correlation coefficient, but those skilled in the art will be aware of other measures of associated that can be used.

In step 143, the plurality of parameters are 'filtered' to remove parameters that have a measure of statistical dependence that is below a threshold value. Where the measure of statistical dependence is a Spearman correlation coefficient, the threshold value can be 0.4, although higher or lower values can be used. The parameters remaining after this filtering (i.e. the parameters for which the measure of statistical dependence is above the threshold value) are referred to herein as a 'second' subset of the plurality of parameters. In subsequent steps of the method in Figure 6 the 'first' subset of parameters described above are derived from this second subset of parameters.

After step 143, in step 145, a second measure of statistical dependence (e.g. a correlation coefficient) is determined for each of the parameters in the second subset of parameters with each of the other parameters in the second subset. In some embodiments, to reduce the number of computations required, the second measure of statistical dependence may only be determined with each of the other parameters in the second subset that has a lower first measure of statistical dependence. In that case, step 145 starts by evaluating the parameter in the second subset that has the highest first measure of statistical dependence, and proceeds with the parameter with the next highest first measure of statistical dependence. Thus, this step evaluates the statistical dependence between a first parameter in the subset with each of the other parameters in the subset, and then repeats that process for a second parameter in the subset, and so on. In particular embodiments, the measure of the statistical dependence is a Spearman's rank correlation coefficient, but those skilled in the art will be aware of other measures of statistical dependence that can be used.

Since the second measure of statistical dependence indicates how closely a particular parameter is related to another parameter, the second measure of statistical dependence can be used to filter out 'redundant' parameters from the subset that will not contribute much extra information to the estimation of the TUG time. Therefore, in step 147, for any pair of parameters in the second subset that has a second measure of statistical dependence above a second threshold value, the parameter having the lowest first measure of statistical dependence (i.e. the lowest statistical dependence on TUG time) is discarded from the second subset. In the case of a SCC, the second threshold value can be 0.8, although it will be appreciated that this value is merely exemplary.

Once step 147 has been completed for each of the parameters, the remaining ones of the second subset of parameters for the 'first' subset of parameters that are to be used to form the model.

There is therefore provided a way to monitor the mobility of a subject during normal and/or unstructured physical activities to provide a measure of the mobility of the subject. The disclosed techniques enable a standardised mobility measure (the TUG time) to be estimated during normal activities, and particularly allows remote monitoring of functional recovery (for instance after hip replacement surgery or a stroke) and/or provides an objective measure to monitor a subject's progress in response to a certain treatment or intervention (for instance rehabilitation) in an outpatient setting. Similar to the TUG time, the TUG time estimate may be used by a care provider to tailor the duration or intensity of an intervention, to monitor the compliance and engagement of the subject with a specified intervention, and by the subject themselves for self-management and goal-setting. The disclosed techniques also enable healthcare providers to remotely collect a TUG time estimate to optimise clinical decision making and consequently improve quality of care (for instance by limiting the interventions to the time to recover or time to stabilise).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus (2) for assessing the mobility of a subject, the apparatus (2) comprising a processing unit (4) that is configured to:

   obtain measurements of movements of the subject over time;

process the obtained measurements to determine values for a first plurality of parameters; and
combine the determined values for the first plurality of parameters to determine an estimate of the time that the subject would take to complete a timed up-and-go, TUG, test.

2. An apparatus (2) as claimed in claim 1, wherein the measurements of the movements of the subject are measurements of the movements of the subject during activities of daily living of the subject.

3. An apparatus (2) as claimed in claim 1 or 2, wherein the processing unit (4) is configured to combine the values for the first plurality of parameters according to a model to determine the estimate of the time that the subject would take to complete a TUG test.

4. An apparatus (2) as claimed in claim 3, wherein the processing unit (4) is further configured to determine the model by:

obtaining measurements of the movements of a plurality of people over time;
obtaining measurements of the times taken for the plurality of people to complete the TUG test;
processing the obtained measurements of the movements of the plurality of people and obtained measurements of the times taken for the plurality of people to complete the TUG test to determine values for a second plurality of parameters;
evaluating the values for the second plurality of parameters to identify a first subset of the second plurality of parameters that are statistically dependent on the obtained measurements of the times taken for the plurality of people to complete the TUG test, wherein the first subset of the second plurality of parameters is the first plurality of parameters; and
deriving the model for determining an estimate of the time that a subject would take to complete the TUG test based on the first subset of the second plurality of parameters.

5. An apparatus as claimed in claim 4, wherein the processing unit (4) is further configured to evaluate the values for the second plurality of parameters by:

determining a first measure of statistical dependence for each of the parameters in the second plurality of parameters based on a measure of association between the values for each of the second plurality of parameters and the time taken for the plurality of people to complete the TUG test; and
selecting the first subset of the second plurality of parameters as (i) the parameters having a measure of statistical dependence above a threshold value; or (ii) a predetermined number of parameters having the highest measures of statistical dependence.

6. An apparatus (2) as claimed in claim 4, wherein the processing unit (4) is further configured to evaluate the values for the second plurality of parameters by:

determining a first measure of statistical dependence for each of the parameters in the second plurality of parameters based on a statistical dependence between the values for each of the second plurality of parameters and the time taken for the plurality of people to complete the TUG test;
selecting a second subset of the second plurality of parameters as the parameters having a first measure of statistical dependence above a first threshold value;
determining a second measure of statistical dependence for each of the parameters in the second subset of the second plurality of parameters based on a measure of association between the values for each of parameters in the second subset of the second plurality of parameters with each of the other parameters in the second subset of the second plurality of parameters;
for any pair of parameters in the second subset of the second plurality of parameters having a second measure of statistical dependence above a second threshold value, discarding from the second subset the one of the pair of parameters having the lowest first measure of statistical dependence; and
forming the first subset of the second plurality of parameters based on the remaining ones of the second subset of the second plurality of parameters.

7. An apparatus (2) as claimed in any of claims 1-6, the apparatus (2) further comprising one or more sensors (12, 14, 16, 18) for measuring movements of the subject over time, and wherein the processing unit (4) is configured to obtain the measurements of the movements of the subject from the one or more sensors (12, 14, 16, 18).

8. A system for assessing the mobility of a subject, the system comprising:

a sensor unit (10) comprising one or more sensors (12, 14, 16, 18) for measuring movements of the subject over time, wherein the sensor unit (10) is configured to generate a sensor signal based on said measured movements of the subject over time; and

an apparatus (2) as claimed in any of claims 1-6, wherein the processing unit (4) is configured to obtain the measurements of the movements of the subject by receiving the sensor signal from the sensor unit (10).

9. A method of assessing the mobility of a subject, the method in a processing unit comprising:

obtaining (101) measurements of the movements of the subject over time;

processing (103) the obtained measurements of the movements to determine values for a first plurality of parameters; and

combining (105) the determined values for the first plurality of parameters to determine an estimate of the time that the subject would take to complete a timed up-and-go, TUG, test.

10. A method as claimed in claim 9, wherein the measurements of the movements of the subject are measurements of the movements of the subject during activities of daily living of the subject.

11. A method as claimed in claim 9 or 10, wherein the step of combining (105) comprises combining the values for the first plurality of parameters according to a model to determine the estimate of the time that the subject would take to complete a TUG test.

12. A method as claimed in claim 11, wherein the method further comprises the steps of:

obtaining (121) measurements of the movements of a plurality of people over time;

obtaining (123) measurements of the times taken for the plurality of people to complete the TUG test;

processing (125) the obtained measurements of the movements of the plurality of people and obtained measurements of the times taken for the plurality of people to complete the TUG test to determine values for a second plurality of parameters;

evaluating (127) the values for the second plurality of parameters to identify a first subset of the second plurality of parameters that are statistically dependent on the obtained measurements of the times taken for the plurality of people to complete the TUG test, wherein the first subset of the second plurality of parameters is the first plurality of parameters; and

deriving (127) the model for determining an estimate of the time that a subject would take to complete the TUG test based on the first subset of the second plurality of parameters.

13. A method as claimed in claim 12, wherein the step of evaluating (127) further comprises evaluating the values for the second plurality of parameters by:

determining (141) a first measure of statistical dependence for each of the parameters in the second plurality of parameters based on a measure of association between the values for each of the second plurality of parameters and the time taken for the plurality of people to complete the TUG test; and

selecting (143) the first subset of the second plurality of parameters as (i) the parameters having a measure of statistical dependence above a threshold value; or (ii) a predetermined number of parameters having the highest measures of statistical dependence.

14. A method as claimed in claim 12, wherein the step of evaluating (127) further comprises evaluating the values for the second plurality of parameters by:

determining (141) a first measure of statistical dependence for each of the parameters in the second plurality of parameters based on a statistical dependence between the values for each of the second plurality of parameters and the time taken for the plurality of people to complete the TUG test;

selecting (143) a second subset of the second plurality of parameters as the parameters having a first measure of statistical dependence above a first threshold value;

determining (145) a second measure of statistical dependence for each of the parameters in the second subset of the second plurality of parameters based on a measure of association between the values for each of parameters in the second subset of the second plurality of parameters with each of the other parameters in the second subset of the second plurality of parameters;

for any pair of parameters in the second subset of the second plurality of parameters having a second measure of statistical dependence above a second threshold value, discarding (147) from the second subset the one of the pair of parameters having the lowest first measure of statistical dependence; and

forming (149) the first subset of the second plurality of parameters based on the remaining ones of the second subset of the second plurality of parameters.

15. A computer program product comprising a computer readable medium having computer readable code embodied therein, the computer readable code being configured such that, on execution by a suitable computer or processor, the computer or processor is caused to perform the method of any of claims 9-14.

```
                    10                                              2
         12              Accelerometer                      Processing unit        4

         14              Gyroscope                                              8

                                                                       Interface
         16              Air pressure
                         sensor
                                                                            6
         18              Position sensor                   Memory unit
```

Figure 1

101 — Obtain measurements of the movements of the subject over time

103 — Process the measurements to determine values for a first plurality of parameters

105 — Combine the values to determine a TUG time estimate

Figure 2

121 — | Obtain measurements of the movements of a plurality of people over time |

123 — | Obtain measurements of the times taken for the plurality of people to complete the TUG test |

125 — | Process the measurements of the movements to determine values for a plurality of parameters |

127 — | Evaluate the values for the plurality of parameters to identify a first subset of the plurality of parameters that are statistically dependent on the measurements of the times taken for the plurality of people to complete the TUG test |

129 — | Derive the model for determining an estimate of the time that a subject would take to complete the TUG test based on the first subset of the plurality of parameters |

Figure 3

Figure 4

17

Figure 5

141 — Determine a first measure of statistical dependence for each of the parameters based on a measure of association between the values for each of the plurality of parameters and the time taken for the plurality of people to complete the TUG test

143 — Select a subset of parameters as the parameters having a first measure of statistical dependence above a first threshold value

145 — Determine a second measure of statistical dependence for each of the parameters based on a measure of association between the values for each of parameters in the subset of the plurality of parameters with each of the other parameters in the subset of the plurality of parameters

147 — For any pair of parameters in the subset having a second measure of statistical dependence above a second threshold value, discard from the subset the one of the pair of parameters having the lowest first measure of statistical dependence

149 — Form the first subset of the plurality of parameters based on the remaining ones of the subset of the plurality of parameters

Figure 6

Europäisches
Patentamt

European
Patent Office

Office européen
des brevets

# EUROPEAN SEARCH REPORT

Application Number

EP 17 19 4125

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 706 949 B2 (JOVANOV EMIL [US] ET AL) 18 July 2017 (2017-07-18) * column 2, line 30 - column 6, line 14 * | 1-15 | INV. A61B5/11 A61B5/00 |
| X | US 2013/023798 A1 (GREENE BARRY R [IE] ET AL) 24 January 2013 (2013-01-24) * paragraphs [0025] - [0034], [0068] - [0088] * | 1-15 | |
| Y | EP 2 264 988 A1 (DEUTSCHE TELEKOM AG [DE]; UNIV BERLIN TECH [DE]) 22 December 2010 (2010-12-22) * paragraphs [0026], [0033] - [0040] * | 1-15 | |
| Y | J DIANE PODSIADLO ET AL: "The Timed "Up & Go": A Test of Basic Functional Mobility for Frail Elderly Persons", LAGS, vol. 39, 1 January 1991 (1991-01-01), pages 142-148, XP055456681, * abstract * * /* Discussion p. 145-147 */ * | 1-15 | |
| A | MATTHEW A. BRODIE ET AL: "Comparison between clinical gait and daily-life gait assessments of fall risk in older people : Remote vs clinical fall-risk assessments", GERIATRICS AND GERONTOLOGY INTERNATIONAL, vol. 17, no. 11, 8 February 2017 (2017-02-08), pages 2274-2282, XP055456810, AU ISSN: 1444-1586, DOI: 10.1111/ggi.12979 * abstract * * /* Methods p.2275 -2277 */ /* Results p. 2277 - 2279 */ /* Discussion p. 2280-2281 */ * | 1-15 | TECHNICAL FIELDS SEARCHED (IPC) A61B |

The present search report has been drawn up for all claims

| Place of search | Date of completion of the search | Examiner |
|---|---|---|
| Berlin | 7 March 2018 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 4125

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2018

| Patent document cited in search report | | Publication date | Patent family member(s) | Publication date |
|---|---|---|---|---|
| US 9706949 | B2 | 18-07-2017 | NONE | |
| US 2013023798 | A1 | 24-01-2013 | NONE | |
| EP 2264988 | A1 | 22-12-2010 | NONE | |

EPO FORM P0459

For more details about this annex : see Official Journal of the European Patent Office, No. 12/82